# EUROPÄISCHE PATENTANMELDUNG

(11) **EP 0 993 805 A1**
(43) Veröffentlichungstag der Anmeldung: **19.04.2000**
(21) Anmeldenummer: 98114430.6
(22) Anmeldetag: 15.10.1998
(51) Int. Cl.: A61B 5/103

(54) **Anordnung zur Vorbeugung gegen thrombotische Embolie**

(71) Anmelder: Medical concept Werbeagentur GmbH, 85748 Garching (DE)
(72) Erfinder: Knips, Jochen, 85375 Neufahrn (DE)

(57) **Zusammenfassung**

Eine Anordnung zur Früherkennung von Beinvenenthrombosen beruht auf der Kombination von Umfangs- und Temperaturmessungen am Bein und kann in Kombination mit medizinischen Kompressionsstrümpfen/strumpfhosen eingesetzt werden.

Mittels dehnungs- und wärmesensibler Meßstreifen werden die Normaldaten gefährdeter Personen erfaßt und in einem Microchip gespeichert. Die Meßstreifen können das Bein ganz oder teilweise umfassen.

Aktuelle Meßdaten werden im entsprechend programmierten Microchip abgeglichen. Bei nicht tolerierten Abweichungen erfolgt Warnung durch optisches und/oder akustisches Signal.

In Kliniken, betreuten Heimen oder im Rahmen telemedizinischer Überwachung wird das Warnsignal zur überwachenden Station oder Person übertragen.

## Beschreibung

Beinvenenthrombosen sind Blutgerinnsel, die meist in den tiefen Beinvenen entstehen und nicht selten vorn Blutstrom bis in die Lunge mitgerissen werden. Der Thrombus verstopft hier ein blutführendes Gefäß und löst so eine lebensgefährdende Lungenembolie aus.

Für die Entstehung dieser Thrombosen kommen drei Ursachen in Frage:
1.) Verlangsamter Rückfluß des venösen Blutes aus den Beinen in Richtung Herzen
2.) Blutgerinnungsstörungen
3.) Schäden an der Veneninnenwand

Besonders gefährdet sind frisch operierte und bettlägerige Personen, Patienten mit Blutgerinnungsstörungen und solche mit venösen Beinleiden.

Weltweit sterben p.a. etwa 1,5 Millionen Personen einer Lungenembolie. Mehr als 10 Million Patienten in den wirtschaftlich entwickelten Staaten leiden unter den Folgen eines postthrombotischen Syndroms, das meist zur dauerhaften Arbeitsunfähigkeit führt.

Als prophylaktische Maßnahmen werden zur Beschleunigung des venösen Rückstroms Kompressionsstrümpfe eingesetzt Patienten mit Blutgerinnungsstörungen werden medikamentös behandelt. Im klinischen Bereich wird die prä- und postoperative prophylaktische Anwendung von Kompression mit medikamentöser Prophylaxe kombiniert.

All diese Maßnahmen verhindern nicht, daß weltweit pro Jahr mehrere Millionen Menschen eine Lungenembolie erleiden. Der Grund hierfür liegt darin, daß es bisher kein effizientes System zur Früherkennung einer Thrombosebildung gibt

Hier ist der Ansatz für TEPYS, mit dem Thrombosen früh erkannt und entweder medikamentös aufgelöst oder operativ entfernt werden können.

Die ersten Anzeichen einer Beinvenenthrombose bestehen unter anderem in Symptomen wie einseitig zunehmendem Beinumfang unterhalb der Thrombose. Diese Umfangsveränderung entsteht dadurch, daß der Rückfluß des Blutes in Richtung Herzen durch den Thrombus behindert wird und ein venöser Blutstau entsteht in der Folge des Status kommt es zu Entzündungen in der Umgebung des Thrombus, die sich durch lokale Erwärmung des Beines bemerkbar machen.

Betroffene und ärztlich erfaßte Personen tragen In der Regel an beiden Beinen Kompressionsstrümpfe oder Kompressionsstrumpfhosen zur Sicherstellung des venösen Kreislaufs.

*I*n diese Kompressionsstrümpfe, bzw- -hosen (1), werden in den Bereichen des Knöchels (2a), der Wade (2b) und des Oberschenkels (2c) jeweils ein dehnungs- und wärmesensibler Meßstreifen (2a,b,c) eingearbeitet vernetzt (3) und zur Datenübertragung, -erfassung und -verarbeitung mit einem Chip (4) verbunden.

Kompressionsstrümpfe und-hosen werden in der Waschmaschine gereinigt Hierzu werden die Meßstreifen und Mikrochips entweder so eingearbeitet, daß sie von Wasser und Wärme nicht beschädigt werden, oder für den Weschvorgang repositionierbar sind.

Die Daten des normalen Beinumfangs und der normalen Temperatur in den beurteilten Beinbereichen der anwendenden Person werden mit dem ersten Anlegen der Kompressionsstrümpfe oder -hosen erfaßt und im Mikrochip (4) gespeichert. Treten während des Tragens der Strümpfe oder Hosen Abweichungen in Form von Zunahme des Umfangs und der Temperatur auf, wird die Person durch ein akustisches und/oder optisches Signal alarmiert, das drahtios an einen Signalgeber (5) übertragen wird.

Im Bereich von Kliniken, Seniorenheimen und medizinisch betreutem Wohnen wird dieses Signal vom Siganlgeber automatisch an die Wachstation übertragen. Werden im Rahmen der erforderlichen Untersuchung zusätzlich Schmerzen, Verfärbungen am Bein oder Atemnot registriert, ist ein Notfall gegeben.

Für alle Thromben, die mit Hilfe von TEPYS erkannt werden bevor sie sich vom Ort der Entstehung lösen in die Lunge wandem gilt, daß sie für den Betroffenen Patienten bei rechtzeitiger Therapie keine Lebensgefährdung mehr mit sich bringen.

Gleichzeitig wird sichergestellt, daß durch Früherkennung und Behandlung die Anzahl und die Ausprägung von postthrombotischen Syndromen deutlich gemindert wird.

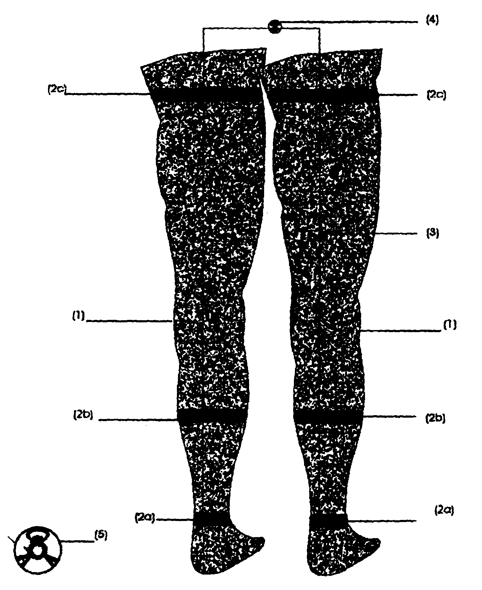

## Patentansprüche

1. Thrombose-Embolie-Prophylaxe-Systeme, die auf der kombinierten Erfassung und Auswertung von Beinumfang und Beintemperatur beruhen.

2. Programmierter Microchip zur Erfassung der von Person zu Person variablen Normaldaten von Beinumfang und Beintemperatur zum Abgleich mit aktuellen Meßdaten und Signalauslösung bei nicht tolerierten Abweichungen.

3. Thrombose-Embolie-Prophylaxe-Systeme, die unter Verwendung von dehnungs- und wärmesensiblen Meßstreifen das Bein ganz oder teilweise umfassen.

4. Thrombose-Embolie-Prophylaxe-Systeme, welche die überwachte Person durch ein optisches und/oder akustisches Signal warnen.

5. Thrombose-Embolie-Prophylaxe-Systeme, welche beim Auftreten von Thrombosen optische und/oder akustische Signale an eine Überwachungsstation weiterleiten.

6. Thrombose-Embolie-Prophylaxe-Systeme, die auf der kombinierten Erfassung und Auswertung von Beinumfang und Beintemperatur beruhen und in Kombination mit der Anwendung von medizinischen Kompressionsstrümpfen oder medizinischen Kompressionsstrumpfhosen eingesetzt werden.
